# EUROPÄISCHE PATENTSCHRIFT

(11) **EP 3 401 312 B1**
(45) Veröffentlichungstag und Bekanntmachung des Hinweises auf die Patenterteilung: **04.09.2019**
(21) Anmeldenummer: 17170003.2
(22) Anmeldetag: 08.05.2017
(51) Int. Cl.: C07D 307/48, C07D 213/89

(54) **VERFAHREN ZUR STABILISIERUNG VON 5 HYDROXYMETHYLFURFURAL (HMF)**
METHOD FOR STABILISATION OF HYDROXYMETHYLFURFURAL (HMF)
PROCÉDÉ DE STABILISATION DE 5-HYDROXYMÉTHYLFURFURAL (HMF)

(43) Veröffentlichungstag der Anmeldung: 14.11.2018
(73) Patentinhaber: AVALON Industries AG, 6304 Zug (CH)
(72) Erfinder: MORTATO, Mariangela, 4051 Basel Stadt (CH); KRAWIELITZKI, Stefan, 6343 Holzhäusern (CH); BADOUX, Francois, 6343 Rotkreuz (CH)
(74) Vertreter: Geitz Truckenmüller Lucht Christ

(56) Entgegenhaltungen:
- EP-A2- 2 450 400
- WO-A1-2007/045886
- WO-A1-2014/158554
- JP-A- 2016 011 264
- KR-A- 20150 006 800
- US-B2- 9 365 531
- VOEST E E ET AL: "An electron paramagnetic resonance study of the antioxidant properties of the nitroxide free radical tempo", FREE RADICAL BIOLOGY AND MEDICINE, ELSEVIER INC, US, Bd. 15, Nr. 6, 1. Dezember 1993 (1993-12-01), Seiten 589-595, XP023523238, ISSN: 0891-5849, DOI: 10.1016/0891-5849(93)90161-M [gefunden am 1993-12-01]

## Beschreibung

Die Erfindung betrifft ein Verfahren zur Stabilisierung von 5-Hydroxymethylfurfural (HMF). Im Besonderen betrifft die Erfindung ein Verfahren zur Vermeidung des Abbaus und der Oligomerbildung von HMF unter Verwendung von sterisch gehinderten Piperidin-N-Oxyl-Radikalen, bevorzugt von 2,2,6,6-Tetramethyl-1-piperidinyloxyl (TEMPO). Weitere Aspekte der Erfindung betreffen ein Stoffgemisch, enthaltend HMF, mindestens ein sterisch gehindertes Piperidin-N-Oxyl-Radikal in einer wirksamen Menge und ein aprotisches Lösungsmittel sowie die Verwendung von sterisch gehinderten Piperidin-N-Oxylen als Stabilisatoren von HMF.

Sterisch gehinderte Piperidin-N-Oxyle haben eine weit verbreitete Anwendung als Stabilisatoren von Kunststoffen und von ethylenisch ungesättigten Monomeren. Die EP 193 772 6 A1 beschreibt beispielsweise N-Oxyle verschiedener Derivate von 2,2,6,6-tetraalkylierten Piperidinen und ihre die Verwendung zur Stabilisierung von Monomeren wie Styrol.

5-Hydroxymethylfurfural (HMF) 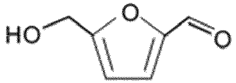 ist ein intramolekulares Dehydratisierungsprodukt von Hexosen und fällt bei der thermischen Zersetzung kohlenhydratreicher Biomasse an. HMF kann besonders umweltschonend durch eine hydrothermale Behandlung wässriger Suspensionen pflanzlicher Biomasse oder wässriger Lösungen von Hexosen wie Fructose bei Temperaturen von 150 bis 250°C und unter Sattdampfdruck gewonnen werden.

Als modere Plattformchemikalie steht HMF im Zentrum einer Reihe industrieller Anwendungen und dient beispielsweise als Vorstufe für die Synthese verschiedener nicht-mineralölbasierter Lösungsmittel, Tenside, Pharmazeutika, Fein- und Spezialchemikalien sowie von Verbindungen zur Herstellung von Polymeren. HMF kommt damit eine Schlüsselrolle beim Wandel von der erdölorientierten Chemie zu einer Chemie auf der Basis von nachwachsenden Rohstoffen zu.

Die Anwendung von HMF ist jedoch bekanntermaßen mit Problemen behaftet. HMF zeigt in Anwesenheit einer ganzen Reihe von Faktoren wie Sauerstoff, Licht, Temperatur oder unter Einfluss von Chemikalien eine Neigung unter Bildung von Oligomeren oder anderen Abbauprodukten zu degenerieren.

Galkin et al. beschreiben in der Fachzeitschrift Angewandte Chemie International Edition (2016 55, 1-6) die Alterung und den Abbau von HMF unter Bildung von Oligomeren.

Die Bildung von Oligomeren in Lösungen von HMF ist beispielweise auch aus der DE 10 2014 112 240 A1 bekannt.

Oligomere sind hierbei auf vielfältige Weise verknüpfte, kurzkettige, lineare oder bei entsprechender Größe auch verzweigte Ketten aus zwei oder mehr HMF-Molekülen. Sie sind unerwünscht, da sie aufgrund ihrer Entstehung eine starke Ähnlichkeit hinsichtlich physikalischer und chemischer Eigenschaften zu HMF aufweisen und zu Schwierigkeiten in der Bearbeitung des HMFs und zur Senkung dessen Reinheit führen. Zudem verringern sie die Effizienz der Verwendung von HMF. Während der Herstellung, der Lagerung, des Transports oder der Bearbeitung ist es jedoch überaus umständlich und schwierig, bisweilen sogar unmöglich, eine entsprechende Exposition des HMFs an der Luft, im Licht oder gegenüber reaktionsfähigen Chemikalien zu vermeiden.

Zur Verringerung der unerwünschten Oligomere von HMF wird in der DE 10 2014 112 240 A1 vorgeschlagen, diese im Anschluss an ihre Bildung aufgrund unterschiedlicher Teilchengröße durch Filtration aus dem HMF zu entfernen. Dies bedarf jedoch eines zusätzlichen Arbeitsschritts.

In der US 9 365 531 B2 wird ebenfalls die Instabilität des HMFs insbesondere bei hohen Temperaturen angesprochen und auf die damit verbundene Problematik der geringen Reinheit von Produkten, die durch Oxidation aus HMF erhalten werden, hingewiesen. Zur Herstellung des Oxidationsprodukts 2,5-Diformylfuran lehrt die US 9 365 531 B2 die Verwendung bestimmter sterisch gehinderter Piperidin-N-Oxyl-Radikale als Katalysatoren, die Teil einer oxidierenden Formulierung sind. Die US 9 365 531 B2 offenbart jedoch weder die Verwendung von sterisch gehinderten Piperidin-N-Oxyl-Radikalen als Stabilisatoren für HMF, noch schlägt sie eine solche vor.

Die Aufgabe der Erfindung bestand darin, die Oligomer-Bildung bei HMF zu verhindern oder zumindest zu vermindern.

Es wurde gefunden, dass sterisch gehinderte Piperidin-N-Oxyl-Radikale überraschend geeignet sind, die Oligomer-Bildung bei HMF zu verhindern bzw. zu vermindern. Darüber hinaus haben die Erfinder festgestellt, dass sterisch gehinderte Piperidin-N-Oxyl-Radikale die Bildung von Furfural, einem Abbauprodukt von HMF, verhindern oder vermindern. Sterisch gehinderte Piperidin-N-Oxyl-Radikale dämmen somit die Degeneration des HMFs effektiv ein und weisen die gewünschten Stabilisatoreigenschaften gegenüber HMF auf.

In einem Aspekt betrifft die Erfindung ein Verfahren zur Stabilisierung von 5-Hydroxymethylfurfural (HMF), das den Zusatz mindestens eines sterisch gehinderten Piperidin-N-Oxyl-Radikals in einer wirksamen Menge umfasst.

Bei dem mindestens einen Piperidin-N-Oxyl-Radikal handelt es sich um ein sogenanntes stabiles Nitroxylradikal, das sich vom Piperidin ableitet und worin sämtliche Substituenten in α-Position zum Stickstoffatom von Wasserstoff verschieden sind. Das mindestens eine Piperidin-N-Oxyl-Radikal kann auch immobilisiert an einen geeigneten Träger vorliegen. Träger können insbesondere organische oder anorganische Partikel sein, wie beispielsweise organische oder anorganische Polymere oder magnetische und nicht-magnetische Metalle, Metalloxide oder Hybrid-Partikel aus verschiedenen Materialien, wie beispielsweise SiO₂-beschichtete Metallpartikel, an die das mindestens eine Piperidin-N-Oxyl-Radikal mittelbar oder unmittelbar gebunden ist.

Durch den Zusatz einer wirksamen Menge mindestens eines sterisch gehinderten Piperidin-N-Oxyl-Radikals zu dem HMF, kann man die Sauerstoff-, Licht-, Temperaturbeständigkeit, die physikalische und/oder chemische Beständigkeit von HMF erhöhen. Eine wirksame Menge an sterisch gehindertem Piperidin-N-Oxyl-Radikal verhindert beziehungsweise vermindert den Abbau und/oder die Oligomerbildung des HMFs und stabilisiert somit das HMF. Ohne sich an eine Theorie binden zu wollen, wird davon ausgegangen, dass die Vermeidung der Furfural-Bildung bereits einen entscheidenden Beitrag zur Stabilisierung des HMF leistet.

Der Zusatz einer wirksamen Menge mindestens eines sterisch gehinderten Piperidin-N-Oxyl-Radikals kann bedeuten, dass ein sterisch gehindertes Piperidin-N-Oxyl-Radikal oder mehrere sterisch gehinderte Piperidin-N-Oxyl-Radikale dem HMF in einer wirksamen Menge zugesetzt werden. Sterisch gehinderte Piperidin-N-Oxyl-Radikale als Stabilisatoren von HMF können dem erfindungsgemäß zu stabilisierenden HMF vor, während oder nach der Herstellung, der Lagerung, der physikalischen und/oder chemischen Bearbeitung und/oder dem Transport zugesetzt werden.

Das zu stabilisierende HMF kann im Wesentlichen rein vorliegen. Es kann jedoch ebenso in einem Gemisch mit weiteren Verbindungen und/oder Lösungsmitteln enthalten sein. Das HMF kann beispielsweise als kristalliner oder amorpher Feststoff vorliegen. Kristalline Formen umfassen mehr oder weniger reine Formen, wie sie im Allgemeinen bei der Herstellung von HMF gewonnen werden. Lösungen von HMF sind in der Regel solche, die bei der Herstellung von HMF in wässriger Lösung, wie sie beispielsweise bei der Dehydratisierung von Hexosen entstehen, oder als Ergebnis einer Extraktion von (Roh-)HMF aus einer wässrigen Lösung mit organischen Lösungsmitteln anfallen. In den Lösungen liegt das zu stabilisierende HMF üblicherweise im Wesentlichen vollständig gelöst vor. Die erfindungsgemäße Stabilisierung empfiehlt sich jedoch beispielsweise auch bei Dispersionen. Der Übergang zwischen gelöster und dispergierter Form kann hierbei fließend sein, so dass in der vorliegenden Erfindung diesbezüglich nicht unterschieden werden soll. Erfindungsgemäß kann man auch sogenannte ölige Lösungen von HMF behandeln, die HMF in hohen Konzentrationen enthalten, beispielsweise nach dem Abdampfen oder Abfiltrieren von Lösungsmitteln. Darüber hinaus schließt das erfindungsgemäße Verfahren auch solche Lösungen ein, die durch Lösen von mehr oder weniger reinem, festen HMF in geeigneten Lösungsmitteln erhalten werden.

Zur Stabilisierung des HMFs werden die Piperidin-N-Oxyl-Radikale dem HMF erfindungsgemäß zugesetzt, zweckmäßigerweise durch Vermischen, gegebenenfalls unter Rühren. Das mindestens eine sterisch gehinderte Piperidin-N-Oxyl-Radikal kann in das HMF durch verschiedene Verfahren eingebracht werden, wie Trockenvermischung in Form von Pulver oder Naßvermischen in Form von Lösungen. Das mindestens eine sterisch gehinderte Piperidin-N-Oxyl-Radikal kann als solches oder in Form einer Lösung in einem geeigneten Lösungsmittel eingesetzt werden. Das Piperidin-N-Oxyl-Radikal kann auf einmal, portionsweise oder kontinuierlich eingebracht werden. Werden dem zu stabilisierenden HMF mehrere Piperidin-N-Oxyl-Radikale zugesetzt, können diese zeitlich nacheinander, gleichzeitig oder auch bereits vorgemischt zugesetzt werden.

Nach einer vorteilhaften Ausgestaltung der Erfindung umfasst das zu stabilisierende HMF eine Lösung von HMF. Aufgrund seiner Struktur, wie bereits oben gezeigt, weist HMF eine Löslichkeit in einer breiten Palette unterschiedlicher wässriger und organischer protischer und aprotischer Lösungsmittel auf. Lösungsmittel kann jedes Lösungsmittel sein, welches das HMF weitestgehend löst und mit der vorgesehenen Verwendung des HMFs kompatibel ist. Es liegt dabei in der allgemeinen Routine des Fachmanns die sterisch gehinderten Piperidin-N-Oxyl-Radikale hinsichtlich ihrer Löslichkeit auf die jeweilige oder jeweiligen HMF-Lösungen abzustimmen. Bevorzugt besteht das Lösungsmittel aus Wasser, Methanol, Ethanol, Aceton, Ethylacetat, Dimethylformamid, Ether, Dichlormethan, Trichlormethan sowie aus Mischungen hiervon, soweit kompatibel. Besonders bevorzugt umfasst die stabilisierte HMF-Lösung Dichlormethan und/ oder Trichlormethan. In diesen Lösungsmitteln zeigt HMF eine sehr gute Löslichkeit, das HMF wird dabei durch das Lösungsmittel nicht destabilisiert und das Lösungsmittel reagiert nicht mit dem mindestens einen Piperidin-N-Oxyl-Radikal, um seine stabilisierende Wirkung zu verringern oder gar aufzuheben.

Nach einer weiteren vorteilhaften Ausgestaltung der Erfindung liegt das HMF in der Lösung in einer Konzentration von 40 bis 70 Gew.%, bevorzugt in einer Konzentration von 50 bis 60 Gew.%, bezogen auf das Gesamtgewicht der Lösung, vor.

Nach der Erfindung ist das sterisch gehinderte Piperidin-N-Oxyl-Radikal ein solches der allgemeinen Formel I 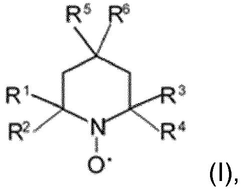 worin
R¹, R², R³ und R⁴ jeweils unabhängig voneinander C₁ - C₆-Alkyl bedeuten, und
R⁵ und R⁶ jeweils unabhängig voneinander für H, Hydroxy, C₁ - C₆-Alkyl, C₁ - C₆-Alkoxy oder Amino stehen.

Bei einem besonders bevorzugten sterisch gehinderten Piperidin-N-Oxyl-Radikal der allgemeinen Formel I bedeuten R¹, R², R³, R⁴ jeweils C₁-Alkyl (Methyl) und R⁵ und R⁶ stehen jeweils für H. Das unter dem Namen 2,2,6,6-Tetramethyl-1-piperidinyloxyl (TEMPO) bekannte Piperidin-N-Oxyl-Radikal wird durch die Formel II 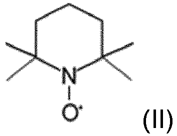 dargestellt. TEMPO wird bevorzugt zur Stabilisierung einer Lösung von HMF eingesetzt. Besonders bevorzugt wird das TEMPO zur Stabilisierung einer Lösung von HMF in einem organischen Lösungsmittel eingesetzt. Das HMF in dem organischen Lösungsmittel kann das Ergebnis einer Extraktion von HMF aus einer wässrigen Lösung sein. Das organische Lösungsmittel kann auch ein Gemisch geeigneter Lösungsmittel sein. Bevorzugt umfasst das organische Lösungsmittel ein aprotisches Lösungsmittel, also ein Lösungsmittel in dem kein feststellbarer Protonenaustausch erfolgt. Die Kombination aus organischem aprotischem Lösungsmittel und TEMPO ist besonders vorteilhaft, da hierdurch eine überraschend hohe Stabilität des HMF erzielt wird. Besonders bevorzugt umfasst das organische Lösungsmittel Dichlormethan und/oder Trichlormethan.

Nach einer besonderen Ausführungsform ist das sterisch gehinderte Piperidin-N-Oxyl-Radikal der allgemeinen Formel I über R⁵ und/oder R⁶ mit einer polymeren Struktur verknüpft. Eine Vielzahl derartiger polymerimmobilisierter sterisch gehinderter Piperidin-N-Oxyl-Radikale ist in der Fachliteratur beschrieben. Beispielsweise beschreiben A. Bogdan und D.T. McQuade in Beilstein Journal of Organic Chemistry 2009, 5, No. 17 die Herstellung eines mit 2,2,6,6-Tetramethyl-1-piperidinyloxyl funktionalisierten Polymerharzes. Weitere Beispiele sind mit 2,2,6,6-Tetramethyl-1-piperidinyloxyl funktionalisierte SiO₂-Nanopartikel (vgl. RSC Adv., 2013, 3, 9752) oder Polyimide (J. Photopolym. Sci. Technol. 27, 2, 2014), Polystyrol- und Polyacrylsäure-geträgertes 2,2,6,6-Tetramethyl-1-piperidinyloxyl sowie PIPO (Polyamin-Immobilisiertes Piperidinyloxyl). Es ist möglich, 2,2,6,6-Tetramethyl-1-piperidinyloxyl und seine Derivate hierbei beispielsweise durch sogenannte Click-Chemie oder durch Polymerisation mit der polymeren Struktur zu verknüpfen. Die Verknüpfung des mindestens einen sterisch gehinderten Piperidin-N-Oxyl-Radikals der allgemeinen Formel I mit einer polymeren Struktur führt dazu, dass ein polymerimmobilisiertes sterisch gehindertes Piperidin-N-Oxyl-Radikal gebildet wird, welches in der Anwendung leicht wieder aus dem HMF entfernt werden kann. Dies ist insbesondere vorteilhaft, da das Piperidin-N-Oxyl-Radikal wiedergewonnen und erneut verwendet werden kann.

Nach einer weiteren vorteilhaften Ausgestaltung der Erfindung wird das zu stabilisierende HMF mit dem mindestens einen sterisch gehinderten Piperidin-N-Oxyl-Radikal in einem molaren Verhältnis von 800:1 bis 1:800 versetzt. Die wirksame Menge an erfindungsgemäß einzusetzenden sterisch gehinderten Piperidin-N-Oxyl-Radikal kann je nach Bedingungen und Konstitution des zu stabilisierenden HMFs in einem weiten Bereich variieren. Aufgrund der unerwartet hohen Wirksamkeit wird häufig auch schon mit sehr wenig sterisch gehindertem Piperidin-N-Oxyl-Radikal der angestrebte stabilisierende Effekt erreicht.

Bevorzugt wird das zu stabilisierende HMF mit dem mindestens einen sterisch gehinderten Piperidin-N-Oxyl-Radikal in einem molaren Verhältnis von 500:1 bis 100:1, besonders bevorzugt von 450:1 bis 150:1 versetzt. Weiter bevorzugt ist ein molares Verhältnis von 420:1 bis 380:1 oder 220:1 bis 160:1. Auch molare Verhältnisse von über 500:1 können erfindungswesentlich sein. Das Versetzen des HMFs mit mindestens einem sterisch gehinderten Piperidin-N-Oxyl-Radikal in derartigen molaren Verhältnissen ist insbesondere vorteilhaft, wenn eine Lösung von HMF kurzzeitig, das bedeutet für Minuten, Stunden oder auch tageweise, stabilisiert werden soll, wie dies bei einer physikalischen und/oder chemischen Behandlung von HMF häufig gefordert wird. Aufgrund der geringen Menge an sterisch gehindertem Piperidin-N-Oxyl-Radikal ist die Stabilisierung darüber hinaus sehr kostengünstig.

Ebenfalls bevorzugt wird das zu stabilisierende HMF mit dem mindestens einen sterisch gehinderten Piperidin-N-Oxyl-Radikal in einem molaren Verhältnis von 4:1 bis 10:1, besonders bevorzugt von 4,5:1 bis 6:1 versetzt. Das Versetzen des HMFs mit mindestens einem sterisch gehinderten Piperidin-N-Oxyl-Radikal in derartigen molaren Verhältnissen ist insbesondere vorteilhaft, wenn eine Lösung von HMF längerfristig, das bedeutet für mehrere Tage, Wochen, Monate oder Jahre, stabilisiert werden soll, wie dies bei einer Lagerung von HMF erwünscht ist.

Nach einer weiteren vorteilhaften Ausgestaltung der Erfindung wird dem HMF neben dem mindestens einen sterisch gehinderten Piperidin-N-Oxyl-Radikal zumindest eine weitere stabilisierende Verbindung zugesetzt. Es ist beispielsweise möglich, dem HMF zusätzlich Antioxidatien zuzusetzen. Diese können dem HMF unabhängig von den sterisch gehinderten Piperidin-N-Oxyl-Radikalen zugegeben werden oder zusammen mit diesen in einer Formulierung.

Weitere Aspekte der Erfindung betreffen ein Stoffgemisch, enthaltend HMF und mindestens ein sterisch gehindertes Piperidin-N-Oxyl-Radikal in einer wirksamen Menge sowie die Verwendung von sterisch gehinderten Piperidin-N-Oxylen als Stabilisatoren von HMF.

Sämtliche Ausführungen zu dem erfindungsgemäßen Verfahren in der vorliegenden Beschreibung mit Bezug auf das zu stabilisierende HMF und/oder die Piperidin-N-Oxyl-Radikale und/oder weiteren Komponenten, gelten selbstverständlich entsprechend für das erfindungsgemäße Stoffgemisch und die erfindungsgemäße Verwendung.

Das erfindungsgemäße Stoffgemisch enthält HMF, eine wirksame Menge mindestens eines sterisch gehinderten Piperidin-N-Oxyl-Radikals der allgemeinen Formel I 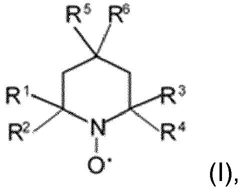 worin R1 , R2 , R3 und R4 die vorstehend beschriebene Bedeutung haben, und R5 und R6 jeweils unabhängig voneinander für H, C1 - C6 -Alkyl, C1 - C6 -Alkoxy oder Amino stehen und ein aprotisches Lösungsmittel.

Das Stoffgemisch ist durch das erfindungsgemäße Verfahren erhältlich, wie in der vorliegenden Beschreibung ausgeführt.

Das aprotische Lösungsmittel ist mit dem Vorteil verbunden, dass das HMF dabei durch das Lösungsmittel nicht destabilisiert wird und dass das Lösungsmittel nicht mit dem mindestens einen Piperidin-N-Oxyl-Radikal reagiert, um seine stabilisierende Wirkung zu verringern oder gar aufzuheben. Das aprotische Lösungsmittel ist Dichlormethan oder Trichlormethan.

Ebenfalls bevorzugt ist das mindestens eine sterisch gehinderte Piperidin-N-Oxyl-Radikal TEMPO.

Die nachfolgenden Beispiele dienen lediglich der Erläuterung der Erfindung und sollen diese in keiner Weise beschränken.

### BEISPIELE

### Beispiel 1

### HMF-Abnahme und Oligomer-Bildung mit und ohne TEMPO während der Lagerung einer HMF-Lösung

Bei der unter Beispiel 1 zusammengefassten Versuchsserie wurde die Abnahme von HMF und die Bildung von Oligomeren in Abhängigkeit der Zeit mit und ohne Stabilisator verglichen. Hierzu wurde kristallines HMF (≤ 97% Reinheit) in Dichlormethan (DCM) gelöst. Die Konzentration des HMF betrug 54 Gew. %, bezogen auf das Gesamtgewicht der Lösung. Ein Teil der Lösung wurde für einen Monat bei Raumtemperatur (RT) sowie unter Tageslicht- und Lufteinfluss gelagert. Zudem wurde eine zweite Lösung von kristallinem HMF (≤ 97% Reinheit) mit einer Konzentration von 53 Gew. % in Dichlormethan (DCM) hergestellt. Der zweiten Lösung wurde TEMPO als Stabilisator in einer Konzentration von 7,7 Gew. %, bezogen auf das Gesamtgewicht der Lösung, zugefügt. Die zweite Lösung, enthaltend TEMPO als Stabilisator, wurde unter den identischen Bedingungen bei Raumtemperatur (RT) und unter Tageslichteinfluss an der Luft gelagert. Um darüber hinaus den gleichzeitigen Einfluss von Licht und Temperatur darzustellen, wurde ein Teil der HMF-Lösung ohne Stabilisator im Dunkeln bei 4°C unter Lufteinfluss ebenfalls für einen Monat aufbewahrt. Während der Lagerung wurde in regelmäßigen Abständen die Konzentration von HMF in jeder der Lösungen durch Hochleistungsflüssigkeitschromatographie (HPLC) bei 280 nm gemessen. In dem gewählten HPLC-System betrug die Retentionszeit der Oligomere zwischen 17 und 19 Minuten. Durch Integration der Flächen unter den für HMF und die Oligomere in dem Chromatogramm der HPLC erhaltenen Peaks wurden die Mengenanteile von HMF und Oligomeren zu den Messzeitpunkten bestimmt.

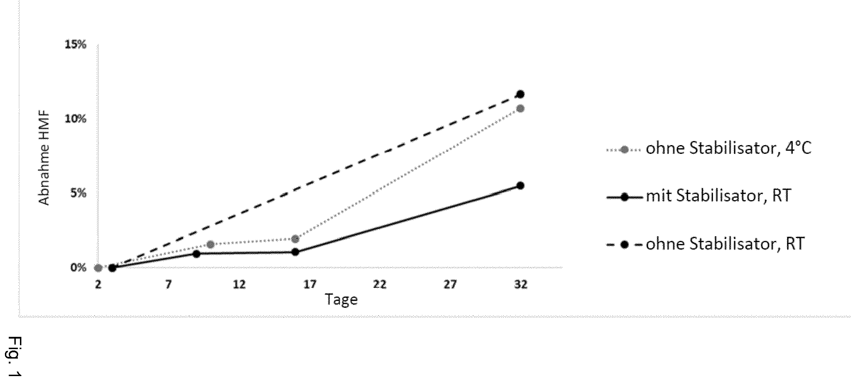

In der Figur 1 der Zeichnung ist die beobachtete Abnahme an HMF in [%] in Abhängigkeit der Zeit graphisch dargestellt. Aus der Figur 1 ist zu entnehmen, dass TEMPO als Stabilisator die Abnahme an HMF in DCM bei Raumtemperatur vermindert. Zudem konnte beobachtet werden, dass die HMF-Abnahme mit TEMPO als Stabilisator bei Raumtemperatur geringer war, als die Abnahme an HMF in der Lösung ohne Stabilisator, die bei 4°C im Dunkeln gelagert wurde. TEMPO ist ein effektiver Inhibitor der Oligomer-Bildung während der Lagerung.

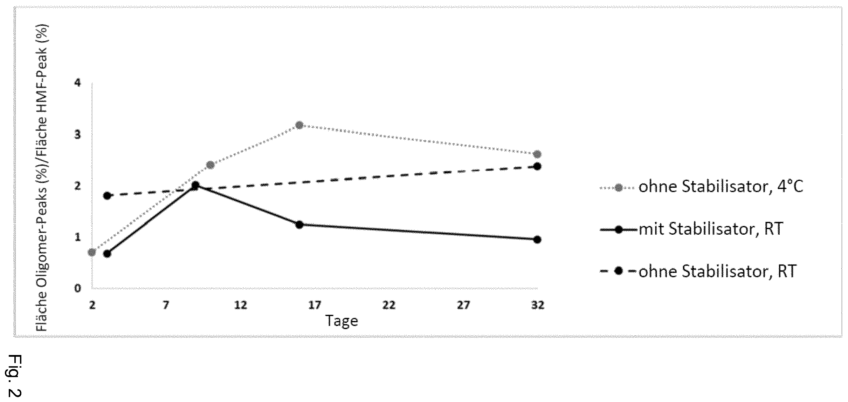

In der Figur 2 ist die mit der Abnahme an HMF einhergehende Oligomer-Bildung in Abhängigkeit der Zeit anhand des Verhältnisses der bei der HPLC ermittelten Fläche der Oligomer-Peaks zu der Fläche des HMF-Peaks dargestellt. Aus Figur 2 wird ersichtlich, dass die Entwicklung der Oligomer-Bildung den stabilisierenden Effekt von TEMPO bestätigt. In Figur 2 ist bei der bei Raumtemperatur gelagerten Lösung mit Stabilisator und bei der bei 4°C gelagerten Lösung eine nach 17 Tagen einsetzende Abnahme der Oligomere zu beobachten. Diese Abnahme ist darauf zurückzuführen, dass sich die Oligomere zu größeren Aggregaten zusammenlagern, welche als unlösliches Sediment durch Filtration vor der Durchführung der HPLC entfernt wurden.

### Beispiel 2

### Oligomer-Bildung mit und ohne TEMPO im Rotationsverdampfer

Beispiel 2 zeigt eine Versuchsserie, die die Bildung von Oligomeren mit und ohne Stabilisator während einer Konzentrierung von HMF-Lösungen in Dichlormethan (DCM) mittels eines Rotationsverdampfers vergleicht.

Hierzu wurden folgende Lösungen hergestellt:
- Lösung A: Lösung von kristallinem HMF in DCM
   Zur Herstellung der Lösung A wurde kristallines HMF (≤ 97% Reinheit) in Dichlormethan (DCM) gelöst.
- Lösung B: Prozesswasser-Extrakt von HMF in DCM
   Zur Herstellung der Lösung B wurde Prozesswasser aus einer hydrothermalen Behandlung von Kohlenhydraten mit DCM extrahiert. Der das HMF enthaltende Extrakt wird als Lösung B bezeichnet.

Die Lösungen A und B wurden in einem Rotationsverdampfer bei 25°C unter Konzentrierung des HMFs eingeengt. Wie die nachfolgende Tabelle 1 zeigt, wurde den Lösungen vor der Konzentrierung im Rotationsverdampfer TEMPO als Stabilisator zugefügt. Vergleichswerte wurden mit den entsprechenden Lösungen ohne TEMPO generiert.

Die Konzentrationen des HMF eingangs der Konzentrierung (Konzentration HMF_IN, Spalte 2) und nach Beenden der Konzentrierung (Konzentration HMF_FIN, Spalte 4) sowie die zugefügten Mengen an TEMPO als Stabilisator (Spalte 3) und die Ergebnisse der Versuchsserie können der Tabelle 1 entnommen werden.

**Tabelle 1**

| | **Konzentration HMF_IN in DCM (mg/l)** | **Menge TEMPO (mM)** | **Konzentration HMF_FIN in DCM (mg/l)** | **Anzahl Oligomer-Peaks** | **Fläche Oligomer-Peaks (%)** | **Fläche Oligomer-Peaks (%)/ Fläche 5-HMF-Peak (%)** | **Fläche 5-HMF-Peak (%)** |
|---|---|---|---|---|---|---|---|
| **Lösung A: Kristallines 5-HMF gelöst in DCM** | 131578 | 0 | 745935 | 2 | 3.16 | 3.29 | 95.9 |
| **Lösung A: Kristallines 5-HMF gelöst in DCM** | 131578 | 5 | 685895 | 3 | 0.53 | 0.54 | 97.7 |
| **Lösung B: 5-HMF mit DCM aus Prozesswasser extrahiert** | 356615 | 0 | 909629 | 10 | 6.52 | 7.60 | 85.8 |
| **Lösung B: 5-HMF mit DCM aus Prozesswasser extrahiert** | 356615 | 5 | 982261 | 8 | 2.47 | 2.74 | 90 |

Die in Tabelle 1 zusammengefassten Ergebnisse zeigen, dass der Anteil an HMF-Oligomeren im Verhältnis zu HMF in den Lösungen deutlich geringer war, denen vor der Konzentrierung im Rotationsverdampfer TEMPO zugemischt wurde (Spalte 7). Generell konnte gezeigt werden, dass die Oligomer-Menge in den mit TEMPO behandelten Lösungen nach der Konzentrierung deutlich geringer ist als in den unbehandelten Lösungen. Im Einklang damit ist die Menge an HMF in den mit TEMPO behandelten Lösungen höher als in den unbehandelten Lösungen. TEMPO ist ein effektiver Inhibitor der Oligomer-Bildung während der physikalischen Bearbeitung des HMFs durch Konzentrierung im Rotationsverdampfer.

### Beispiel 3

### Abbau zu Furfural mit und ohne TEMPO im Rotationsverdampfer

In der Lösung A mit und ohne TEMPO aus Beispiel 2 wurde nach der im Beispiel 2 beschriebenen Konzentrierung im Rotationsverdampfer die Konzentration an Furfural bestimmt. Das Ergebnis ist in Tabelle 2 dargestellt.

**Tabelle 2**

| | **TEMPO (mM)** | **Konzentration Furfural_FIN in DCM (mg/l)** |
|---|---|---|
| **Lösung A: Kristallines 5-HMF gelöst in DCM** | 0 | 653 |
| **Lösung A: Kristallines 5-HMF gelöst in DCM** | 5 | 383 |

Wie aus Tabelle 2 ersichtlich, ist die Konzentration in der mit TEMPO als Stabilisator behandelten Lösung A deutlich geringer, als in der ohne TEMPO konzentrierten Lösung. TEMPO ist ein effektiver Inhibitor des Abbaus von HMF zu Furfural während der physikalischen Bearbeitung des HMFs durch Konzentrierung im Rotationsverdampfer.

Die Beispiele zeigen die unerwartete Stabilisierungswirkung des Piperidin-N-Oxyl-Radikals TEMPO, auch wenn es nur in geringen Mengen zugesetzt wird.

Weitere Vorteile und vorteilhafte Ausgestaltungen sind den Ansprüchen zu entnehmen.

Sämtliche Merkmale der Erfindung können sowohl einzeln als auch in beliebiger Kombination miteinander erfindungswesentlich sein.

## Patentansprüche

1. Verfahren zur Stabilisierung von 5-Hydroxymethylfurfural (HMF), das den Zusatz mindestens eines sterisch gehinderten Piperidin-N-Oxyl-Radikals der allgemeinen Formel I 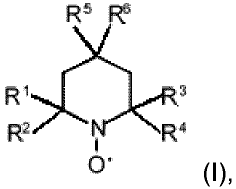 worin
R¹, R², R³ und R⁴ jeweils unabhängig voneinander C₁ - C₆-Alkyl bedeuten, und
R⁵ und R⁶ jeweils unabhängig voneinander für H, Hydroxy, C₁ - C₆-Alkyl, C₁ - C₆-Alkoxy oder Amino stehen,
in einer wirksamen Menge umfasst.

2. Verfahren nach Anspruch 1, wobei das zu stabilisierende HMF eine Lösung von HMF umfasst.

3. Verfahren nach Anspruch 2, wobei das zu stabilisierende HMF in der Lösung in einer Konzentration von 40 bis 70 Gew.%, bevorzugt in einer Konzentration von 50 bis 60 Gew.%, bezogen auf das Gesamtgewicht der Lösung, vorliegt.

4. Verfahren nach einem der vorhergehenden Ansprüche, wobei das sterisch gehinderte Piperidin-N-Oxyl-Radikal 2,2,6,6-Tetramethyl-1-piperidinyloxyl (TEMPO) ist.

5. Verfahren nach einem der vorhergehenden Ansprüche, wobei das sterisch gehinderte Piperidin-N-Oxyl-Radikal der allgemeinen Formel I über R⁵ und/oder R⁶ mit einer polymeren Struktur verknüpft ist.

6. Verfahren nach einem der vorhergehenden Ansprüche, wobei das zu stabilisierende HMF mit dem mindestens einen sterisch gehinderten Piperidin-N-Oxyl-Radikal in einem molaren Verhältnis von 800:1 bis 1:800 versetzt wird.

7. Verfahren nach Anspruch 6, wobei ein molares Verhältnis von 500:1 bis 100:1 bevorzugt ist und ein molares Verhältnis von 450:1 bis 150:1 besonders bevorzugt ist.

8. Verfahren nach Anspruch 6, wobei ein molares Verhältnis von 4:1 bis 10:1 bevorzugt ist und ein molares Verhältnis von 4,5:1 bis 6:1 besonders bevorzugt ist.

9. Verfahren nach einem der vorhergehenden Ansprüche, wobei dem zu stabilisierenden HMF neben dem mindestens einen sterisch gehinderten Piperidin-N-Oxyl-Radikal zumindest eine weitere stabilisierende Verbindung zugesetzt wird.

10. Stoffgemisch, hergestellt nach einem der Ansprüche 1 bis 9, enthaltend HMF, eine wirksame Menge mindestens eines sterisch gehinderten Piperidin-N-Oxyl-Radikals der allgemeinen Formel I 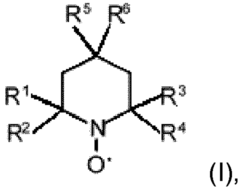 worin
R¹, R², R³ und R⁴ die vorstehend beschriebene Bedeutung haben, und
R⁵ und R⁶ jeweils unabhängig voneinander für H, C₁ - C₆-Alkyl, C₁ - C₆-Alkoxy oder Amino stehen,
und ein aprotisches Lösungsmittel, wobei das aprotische Lösungsmittel Dichlormethan oder Trichlormethan ist.

11. Verwendung von sterisch gehinderten Piperidin-N-Oxyl-Radikalen der allgemeinen Formel I 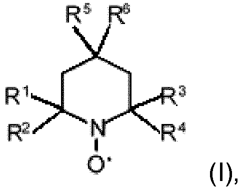 worin
R¹, R², R³ und R⁴ jeweils unabhängig voneinander C₁ - C₆-Alkyl bedeuten, und
R⁵ und R⁶ jeweils unabhängig voneinander für H, Hydroxy, C₁ - C₆-Alkyl, C₁ - C₆-Alkoxy oder Amino stehen,
als Stabilisatoren für HMF.

## Claims

1. A method for stabilizing 5-hydroxymethylfurfural (HMF), comprising the addition of at least one sterically hindered piperidine-*N*-oxyl radical of general formula I 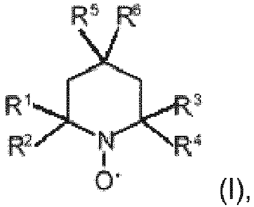 where
R¹, R², R³, and R⁴ each independently stand for C₁-C₆ alkyl, and
R⁵ and R⁶ each independently stand for H, hydroxy, C₁-C₆ alkyl, C₁-C₆ alkoxy, or amino,
in an effective quantity.

2. The method according to Claim 1, wherein the HMF to be stabilized comprises a solution of HMF.

3. The method according to Claim 2, wherein the HMF to be stabilized is present in the solution in a concentration of 40 to 70 wt%, preferably in a concentration of 50 to 60 wt%, based on the total weight of the solution.

4. The method according to one of the preceding claims, wherein the sterically hindered piperidine-*N*-oxyl radical is 2,2,6,6-tetramethyl-1-piperidinyloxyl (TEMPO).

5. The method according to one of the preceding claims, wherein the sterically hindered piperidine-*N*-oxyl radical of general formula I is linked to a polymeric structure via R⁵ and/or R⁶.

6. The method according to one of the preceding claims, wherein the HMF to be stabilized is combined with the at least one sterically hindered piperidine-*N*-oxyl radical in a molar ratio of 800:1 to 1:800.

7. The method according to Claim 6, wherein a molar ratio of 500:1 to 100:1 is preferred, and a molar ratio of 450:1 to 150:1 is particularly preferred.

8. The method according to Claim 6, wherein a molar ratio of 4:1 to 10:1 is preferred, and a molar ratio of 4.5:1 to 6:1 is particularly preferred.

9. The method according to one of the preceding claims, wherein in addition to the at least one sterically hindered piperidine-*N*-oxyl radical, at least one further stabilizing compound is added to the HMF to be stabilized.

10. A mixture prepared according to one of Claims 1 to 9, containing HMF, an effective quantity of at least one sterically hindered piperidine-*N*-oxyl radical of general formula I 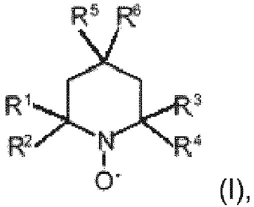 where
R¹, R², R³, and R⁴ have the meanings described above, and
R⁵ and R⁶ each independently stand for H, C₁-C₆ alkyl, C₁-C₆ alkoxy, or amino,
and an aprotic solvent, wherein the aprotic solvent is dichloromethane or trichloromethane.

11. Use of sterically hindered piperidine-*N*-oxyl radicals of general formula I 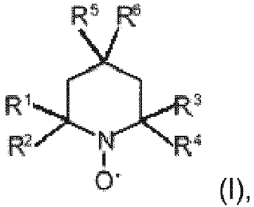 where
R¹, R², R³, and R⁴ each independently stand for C₁-C₆ alkyl, and
R⁵ and R⁶ each independently stand for H, hydroxy, C₁-C₆ alkyl, C₁-C₆ alkoxy, or amino,
as stabilizers for HMF.

## Revendications

1. Procédé de stabilisation d'hydroxy-5-méthylfurfural (HMF) comprenant l'ajout d'au moins un radical pipéridine-N-oxyle à empêchement stérique, de formule générale I 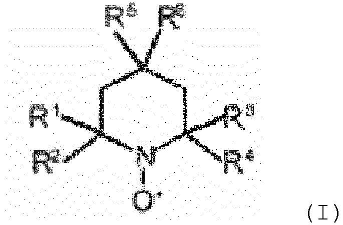
R¹, R², R³ et R⁴ représentant chacun indépendamment un groupe alkyle en C₁ à C₆, et
R⁵ et R⁶ représentant chacun indépendamment un atome de H, un groupe hydroxy, un groupe alkyle en C₁ à C₆, un groupe alcoxy en C₁ à C₆ ou un groupe amino,
dans une quantité efficace.

2. Procédé selon la revendication 1, le HMF à stabiliser comprenant une solution de HMF.

3. Procédé selon la revendication 2, le HMF à stabiliser étant présent dans la solution à une concentration de 40 à 70 % en poids, de préférence à une concentration de 50 à 60 % en poids, sur la base du poids total de la solution.

4. Procédé selon l'une des revendications précédentes, le radical pipéridine-N-oxyle à empêchement stérique étant le radical 2,2,6,6-tétraméthyl-1-pipéridinyloxyle (TEMPO).

5. Procédé selon l'une des revendications précédentes, le radical pipéridine-N-oxyle à empêchement stérique de formule générale I étant combiné à une structure polymérique par le biais du radical R⁵ et/ou R⁶.

6. Procédé selon l'une des revendications précédentes, le HMF à stabiliser étant mélangé à au l'moins un radical pipéridine-N-oxyle à empêchement stérique dans un rapport molaire allant de 800:1 à 1:800.

7. Procédé selon la revendication 6, un rapport molaire allant de 500:1 à 100:1 étant préféré, et un rapport molaire allant de 450:1 à 150:1 étant particulièrement préféré.

8. Procédé selon la revendication 6, un rapport molaire allant de 4:1 à 10:1 étant préféré, et un rapport molaire allant de 4,5:1 à 6:1 étant particulièrement préféré.

9. Procédé selon l'une des revendications précédentes, au moins un autre composé stabilisant étant ajouté au HMF à stabiliser en plus de l'au moins un radical pipéridine-N-oxyle à empêchement stérique,.

10. Mélange préparé selon l'une des revendications 1 à 9, contenant du HMF, une quantité efficace d'au moins un radical pipéridine-N-oxyle à empêchement stérique, de formule générale I 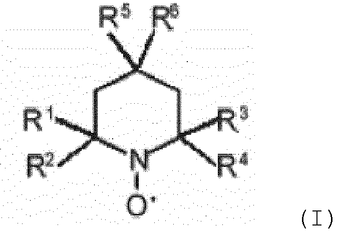
R¹, R², R³ et R⁴ ayant les significations décrites ci-dessus, et
R⁵ et R⁶ représentant chacun indépendamment un atome de H, un groupe alkyle en C₁ à C₆, un groupe alcoxy en C₁ à C₆ ou un groupe amino,
et un solvant aprotique, le solvant aprotique étant le dichlorométhane ou le trichlorométhane.

11. Utilisation de radicaux pipéridine-N-oxyle à empêchement stérique, de formule générale I 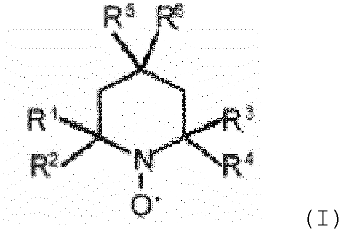
R¹, R², R³ et R⁴ représentant chacun indépendamment un groupe alkyle en C₁ à C₆, et
R⁵ et R⁶ représentant chacun indépendamment un atome de H, un groupe hydroxy, un groupe alkyle en C₁ à C₆, un groupe alcoxy en C₁ à C₆ ou un groupe amino,
comme stabilisant pour le HMF.
